# EUROPÄISCHE PATENTANMELDUNG

(11) **EP 1 887 087 A1**
(43) Veröffentlichungstag der Anmeldung: **13.02.2008**
(21) Anmeldenummer: 07013554.6
(22) Anmeldetag: 11.07.2007
(51) Int. Cl.: C12Q 1/56, G01N 33/86

(54) **Polysaccharid-Peptid-Konjugate zur Verwendung als Thrombinsubstrate**

(30) Priorität: 31.07.2006 DE 102006035899
(71) Anmelder: Dade Behring Marburg GmbH, 35041 Marburg (DE)
(72) Erfinder: Stoellner, Daniela, Dr., 4132 Muttenz (CH); Henckel, Thilo, Dr., 35083 Wetter (DE)

(57) **Zusammenfassung**

Die vorliegende Erfindung betrifft makromolekulare Polysaccharid-Peptid-Konjugate, die einen Peptidanteil enthalten, der C-terminal die Aminosäuresequenz Ala-Gly-Arg enthält, welche von Thrombin gespalten wird.

## Beschreibung

Die Erfindung liegt auf dem Gebiet der Diagnostik, insbesondere der Gerinnungsdiagnostik, und betrifft die Herstellung und Verwendung von Polysaccharid-Peptid-Konjugaten, die einen Peptidanteil enthalten, der eine Aminosäuresequenz enthält, die von Thrombin gespalten wird.

Die Regulation der Blutgerinnung (Hämostase) erfolgt durch das Zusammenspiel verschiedener Aktivatoren, Inhibitoren sowie positiver und negativer Rückkopplungsmechanismen. Defekte in diesem Gefüge können zu einer Dysbalance im Hämostasesystem führen und entweder eine Hämorrhagie oder eine Thrombose zur Folge haben. Thrombin (Faktor IIa, F IIa) ist eine Serinprotease und das zentrale Enzym der plasmatischen Blutgerinnung. Die Hauptfunktion von Thrombin besteht in der Induktion der Fibrinpolymerisation und ist damit wesentlich für die Gerinnselbildung. Die Bildung von Thrombin wird durch Aktivierung des enzymatisch inaktiven Vorläufermoleküls Prothrombin (Faktor II, F II) in Gang gesetzt. Um im Verletzungsfall den Gerinnungsprozess lokal und zeitlich zu begrenzen, erfolgt eine prompte Drosselung der Gerinnung u. a. dadurch, dass das freie Thrombin von inhibitorischen Faktoren wie z. B. Antithrombin oder α₂-Makroglobulin (α₂M) komplexiert und somit inaktiviert wird. Störungen innerhalb der Prozesse, die die Thrombinbildung oder -inhibition regulieren, können zu hyper- bzw. hypokoagulatorischen Zuständen und damit zu pathologischen Gerinnungsstörungen führen. In der Erfassung der Bildung und der Inhibition des Thrombins liegt also eine immense Aussagekraft über den jeweiligen Gerinnungszustand eines Probanden.

Thrombingenerierungsteste sind globale Gerinnungsteste, welche die Bildung und Inhibition von Thrombin in Plasma oder Blut bestimmen. Das einer Probe innewohnende, im Falle von Plasmaproben plasmaeigene Vermögen zur Bildung und Inhibition von enzymatisch aktivem, freiem Thrombin wird auch als endogenes Thrombinpotenzial (ETP) bezeichnet. Da alle biologischen Komponenten, die in einem Untersuchungsmaterial enthalten sind und die Bildung und die Inhibition von Thrombin beeinflussen, das endogene Thrombinpotenzial einer Probe bedingen, eignet sich die ETP-Bestimmung sowohl als globaler Test, mit dem mehrere Komponenten des Hämostasesystems erfasst werden können, als auch zur Überwachung therapeutischer Maßnahmen. Die ETP-Bestimmung ermöglicht die Diagnose hypokoagulatorischer sowie hyperkoagulatorischer Zustände. Weitere Indikationen umfassen angeborene und erworbene Koagulopathien (Hämophilie, Faktorenmangel II, V, VII,VIII, IX, X, XI, disseminierte intravasale Koagulopathie) sowie thrombophile Risikofaktoren (Prothrombinmutation, Faktor V-Leiden, Protein S-, Protein C- und Antithrombinmangel). Auch erworbene und transiente Risikofaktoren wie z. B. Schwangerschaft oder die Einnahme oraler Kontrazeptiva, sowie das Rauchen werden durch erhöhte ETP-Werte reflektiert. Ein weiterer interessanter Aspekt der ETP-Bestimmung ist die Kontrolle von Antikoagulationstherapien. Da die Thrombinbildungsfähigkeit direkt bestimmt wird, wird das Gerinnungspotenzial des Patienten unabhängig von dem oder den eingesetzten Antikoagulantien erfasst. Somit bietet der Parameter ETP auch in den Übergangs- und Einstellphasen der Therapien eine Überwachungsmöglichkeit um Über- und Unterdosierung zu verhindern.

Ursprünglich wurde zur Bestimmung der Thrombingenerierung eine Probe mit einem Prothrombinaktivator versetzt und dem Gemisch wurden in distinkten Zeitabständen Aliquots entnommen. Die Thrombinkonzentration in den einzelnen Aliquots wurde bestimmt, indem die Spaltung eines chromogenen Thrombinsubstrats gemessen wurde. Ein derartige Verfahrensweise, die auch als "Subsampling Method" bekannt ist, ist beispielweise in Hemker et al. (1986) A computer assisted method to obtain the prothrombin activation velocity in whole plasma independent of thrombin decay process. Thromb Haemost. 56 (1) 9-17 auf Seite 10 in dem Absatz "Determination of the Time Course of Amidolytic Activity" beschrieben.

In EP 420 332 B1 ist ein verbessertes Verfahren zur Thrombinbestimmung beschrieben, das eine kontinuierliche Bestimmung der Thrombinkonzentration in dem Reaktionsansatz ermöglicht, so dass auf die oben beschriebene Entnahme mehrerer Aliquots verzichtet werden kann. Wesentlich für die kontinuierliche Bestimmung der Thrombinkonzentration im Reaktionsansatz ist, dass das verwendete Thrombinsubstrat nicht verbraucht ist, bevor die Thrombininhibierung vollendet ist. Die Verwendung von Thrombinsubstraten, die über kinetische Eigenschaften verfügen, so dass sie relativ langsam, aber dennoch proportional zur vorhandenen Thrombinmenge umgesetzt werden, ermöglicht die kontinuierliche Bestimmung der Thrombinkonzentration in einem einzigen Reaktionsansatz. Zur Bestimmung der Thrombingenerierung wird in einer Probe gerinnungsfähigen Blutes oder Plasmas die Umsatzkinetik eines Thrombinsubstrats über die Freisetzung einer detektierbaren Signalgruppe gemessen. Da die Thrombinsubstratkonzentration so eingestellt wird, dass das Substrat im Laufe der Reaktion nicht vollständig aufgebraucht werden kann, verhält sich die Menge an freigesetztem Indikator idealerweise proportional zur enzymatischen Aktivität des im Verlaufe der Gerinnungsreaktion gebildeten Thrombins (siehe auch Hemker, H. C. et al. (1993) Continuous registration of thrombin generation in plasma, its use for the determination of the thrombin potential. Thromb Haemost. 70 (4) 617-624).

In Thrombingenerierungstesten werden üblicherweise kleinmolekulare Thrombinsubstrate eingesetzt, die aus einem Oligopeptid bestehen, an welches eine detektierbare Signalgruppe gekoppelt ist. Durch die enzymatische Aktivität von Thrombin wird die Bindung zwischen Peptid und Signalgruppe hydrolysiert, und die Signalgruppe wird freigesetzt. Über die Messung der Signalstärke kann die Thrombinaktivität quantifiziert werden. Beispiele für Oligopeptidsubstrate, die bekanntermaßen von Thrombin gespalten werden, sind z. B. para-Nitroanilid (pNA)-gekoppelte Peptide der Sequenz Ala-Gly-Arg-pNA, Ala-Arg-pNA, Gly-Arg-pNA oder Pro-Arg-pNA.

Es ist jedoch bekannt, dass mit Thrombinsubstraten, welche eine Molekülgröße von weniger als 8 kD aufweisen, zusätzlich zur physiologisch relevanten Aktivität des freien Thrombins auch die physiologisch irrelevante Aktivität des α₂-Makroglobulin-Thrombin-Komplexes (α₂MT) gemessen wird. Aus der Messung der Menge an freigesetzter Signalgruppe über die Zeit resultiert eine Reaktionskinetik, die trotz fortschreitender und schließlich vollständiger Inhibierung des freien Thrombins keine Plateau phase erreicht, sondern weiterhin ansteigt. Die kleinmolekularen Peptidsubstrate sind offensichtlich in der Lage durch den α₂-Makroglobulin-Thrombin-Komplex (α₂MT) zum aktiven Zentrum des Thrombinmoleküls vorzudringen und werden daher auch von komplexiertem Thrombin noch gespalten. Die Menge an gespaltenem Substrat ist daher nicht strikt proportional zur Menge an freiem Thrombin, sondern ist das Ergebnis der Aktivität von freiem und α₂-Makroglobulin-gebundenem Thrombin. Es sind zwar verschiedene Techniken zur Berechnung der Menge von freiem Thrombin bekannt (z. B. EP 1 669 761 A2, WO 2004/016807 A1), diese sind aber zum Teil relativ aufwändig. Alternative Lösungen, die eine direkte Bestimmung des freien Thrombins anhand der experimentellen Daten ermöglichen, sind daher wünschenswert.

In EP 1 159 448 B1 wird die Verwendung von makromolekularen Ovalbumin-gekoppelten Thrombinsubstraten in einem Thrombingenerierungsassay beschrieben. Da die Ovalbumin-gekoppelten Thrombinsubstrate eine Molekülgröße von mehr als 10 kDa aufweisen, werden sie nicht von α₂-Makroglobulin-gebundenem Thrombin, sondern nur von freiem Thrombin gespalten. Nachteilig ist jedoch, dass es bei der Kopplung von Peptidsubstraten an Ovalbumin zur Herstellung der makromolekularen Substrate häufig technische Probleme gibt. Gelegentlich kommt es dazu, dass die Reaktionslösung hochviskos wird, möglicherweise aufgrund von Ovalbumin-Vernetzungsreaktionen. Die Verwendung Ovalbumin-gekoppelter Thrombinsubstrate ist also aufgrund der Probleme bei der Herstellung dieser Substrate und der damit eingeschränkten Verfügbarkeit der Substrate als unbefriedigend anzusehen. Ein weiterer Nachteil bei der Verwendung von Protein-gekoppelten Makrosubstraten ist, dass sie einem Reaktionsansatz nicht in höheren Konzentrationen zugesetzt werden können, da Präzipitationsreaktionen und damit Trübungen im Reaktionsansatz auftreten können. Dies ist insbesondere für Testverfahren, die mit Hilfe von optischen Methoden ausgewertet werden, nachteilig.

Der vorliegenden Erfindung lag die Aufgabe zugrunde weitere makromolekulare Thrombinsubstrate zur Verfügung zu stellen, die sich für eine Verwendung in einem Thrombingenerierungstest eignen. Bevorzugterweise sollten die makromolekularen Substrate nicht von α₂-Makroglobulin-gebundenem Thrombin gespalten werden. Weiterhin bevorzugt sollten die makromolekularen Thrombinsubstrate über kinetische Eigenschaften verfügen, so dass das Thrombinsubstrat nicht verbraucht ist bevor die Thrombininhibierung in einem kontinuierlichen Thrombingenerierungstest vollendet ist.

Die Lösung dieser Aufgaben besteht in der Bereitstellung der in den Ansprüchen beschriebenen Gegenstände.

Gegenstand der vorliegenden Erfindung ist ein Polysaccharid-Peptid-Konjugat, das aus einem Polysaccharidanteil und einem Peptidanteil zusammengesetzt ist und das ein Molekulargewicht von mehr als 10 kDa aufweist. Der Peptidanteil besteht aus einem mindestens 3 Aminosäuren langen Peptid, dessen C-Terminus die Sequenz Ala-Gly-Arg-R aufweist, wobei R für eine abspaltbare Signalgruppe steht.

Es wurde überraschenderweise gefunden, dass ein Polysaccharid-Peptid-Konjugat, welches am C-Terminus des Peptidanteils die Sequenz Ala-Gly-Arg-R aufweist, aufgrund seiner kinetischen Eigenschaften zur Verwendung als Thrombinsubstrat in einem Thrombingenerierungstest geeignet ist, während andere Polysaccharid-Peptid-Konjugate, die einen anderen thrombinspezifischen Peptidanteil aufweisen, aufgrund ihrer kinetischen Eigenschaften nicht zur Verwendung in einem kontinuierlichen Thrombingenerierungstest geeignet sind. Das erfindungsgemäße Substrat wird von freiem Thrombin gespalten, aber nicht von α₂-Makroglobulin-gebundenem Thrombin. Weiterhin vorteilhaft ist, dass die Herstellung eines erfindungsgemäßen Polysaccharid-Peptid-Konjugats effizienter ist als die problembehaftete Herstellung der aus dem Stand der Technik bekannten Ovalbumin-gekoppelten Thrombinsubstrate.

Polysaccharide bzw. der Polysaccharidanteil eines erfindungsgemäßen Konjugats im Sinne der vorliegenden Erfindung bestehen aus identischen oder unterschiedlichen Monosaccharideinheiten (Homo- bzw. Heteropolysaccharide), die durch glykosidische Bindungen miteinander verbunden sind. Die Struktur des Polysaccaridmoleküls kann linear oder verzweigt sein. Zur Herstellung eines erfindungsgemäßen Polysaccharid-Peptid-Konjugats werden bevorzugterweise Polysaccharide verwendet, die aus Monosaccharid-Einheiten aufgebaut sind, welche vicinale Diole oder Hydroxyl/Aminogruppen oder Hydroxyl/Carbonylgruppen oder Carbonyl/Carbonylgruppen enthalten. Besonders bevorzugte Polysaccharide sind zum Beispiel Dextran, Galactan, Arabinogalactan und Mannan.

Bevorzugterweise hat der Polysaccharidanteil eines erfindungsgemäßen Konjugats eine molare Masse von etwa 10.000 bis etwa 40.000 g/mol, bevorzugt von etwa 12.000 bis etwa 20.000 g/mol, besonders bevorzugt von etwa 15.000 g/mol.

Der Peptidanteil eines erfindungsgemäßen Polysaccharid-Peptid Konjugats besteht aus einem mindestens 3 Aminosäuren langen Peptid, dessen C-Terminus die Sequenz Ala-Gly-Arg-R aufweist, wobei Ala für Alanin, Gly für Glycin, Arg für Arginin und R für eine abspaltbare Signalgruppe steht. Das Peptid kann N-terminal einige weitere Aminosäurereste enthalten. Bevorzugterweise besteht das Peptid insgesamt aus einer Sequenz von 3 bis 5 Aminosäureresten, vorteilhafterweise besteht das Peptid aus nicht mehr als insgesamt 8 Aminosäureresten. Besonders bevorzugt besteht das Peptid aus dem Tripeptid Ala-Gly-Arg.

Bei der C-terminalen Signalgruppe R handelt es sich um eine durch Thrombin abspaltbare Signalgruppe, die nach Abspaltung von dem Arginin-Rest ein detektierbares Signal erzeugt. Bei der Signalgruppe kann es sich beispielsweise um eine chromogene oder fluorogene Gruppe handeln, die mit Hilfe photometrischer Methoden detektiert werden können. Bevorzugte chromogene Signalgruppen sind para-Nitroanilin (pNA) und 5-Amino-2-Nitro-Benzoesäure (ANBA), deren gelbe Farbe bei einer Wellenlänge von λ= 405 nm messbar ist. Eine bevorzugte fluorogene Gruppe ist 7-Amino-4-Methoxy Coumarin (AMC).

Tabelle 1 zeigt die kinetischen Eigenschaften eines erfindungsgemäßen Dextran-β-Ala-Gly-Arg-pNA-Konjugats im Vergleich zu den kinetischen Eigenschaften des ungekoppelten Peptidsubstrats H-β-Ala-Gly-Arg-pNA bzw. der Dextran-gekoppelten Konjugate Dextran-D-CHG-Ala-Arg-pNA und Dextran-D-CHG-Gly-Arg-pNA.

**Tabelle 1**

| | **Km [mM]** | **Vmax [U]** | **Kcat [1/s]** | **Kcat/Km [L/mmol*s]** |
|---|---|---|---|---|
| **H-β-Ala-Gly-Arg-pNA 2AcOH** | **2,2** | **16** | **17,4** | **7,9** |
| **Dextran-β-Ala-Gly-Arg-pNA** | **1,04-1,4** | **19,5** | **21** | **17,5** |
| **Dextran-D-CHG-Ala-Arg-pNA *** | **0,6** | **57** | **167** | **278** |
| **Dextran-D-CHG-Gly-Arg-pNA *** | **0,6** | **60** | **176** | **293** |

| | | | | |
|---|---|---|---|---|
| * D-CHG = D-2-Cyclohexylglycin | | | | |

Um eine kontinuierliche Messung der Thrombingenerierung zu gewährleisten, ist es notwendig, dass das Substrat spezifisch, aber möglichst langsam von Thrombin umgesetzt wird. Die Substratkonzentration (Kₘ), auch Michaelis-Konstante genannt, welche zur Halbsättigung des Enzyms benötigt wird, ist ein Maß für die Substrataffinität von Thrombin. Bei hoher Affinität ist die Substratkonzentration Kₘ klein, das heisst ein kleiner Kₘ-Wert ist Ausdruck einer hohen Affinität für das jeweilige Substrat. Die katalytische Konstante (K_{cat}), auch Wechselzahl genannt, gibt die Umsatzgeschwindigkeit des Enzyms an bzw. die Zahl der Substratmoleküle, welche pro Zeiteinheit durch jedes aktive Zentrum im Enzymmolekül umgesetzt werden. Das Verhältnis K_{cat}/Kₘ wird als katalytische Effizienz bezeichnet. Dieser Wert gilt als Maß für die Substratspezifität, wobei hohe Werte eine hohe Substratspezifität kennzeichnen. Die maximale Reaktionsgeschwindigkeit Vₘₐₓ bezeichnet die maximale Umsatzgeschwindigkeit abhängig von Reaktionsbedingungen (z. B. pH, Temperatur), welche auch durch eine weitere Erhöhung der Substratkonzentration nicht gesteigert werden kann (Sättigungsbereich der Reaktion).

Das ungekoppelte Peptidsubstrat H-β-Ala-Gly-Arg-pNA sowie das Dextrangekoppelte Substrat Dextran-β-Ala-Gly-Arg-pNA erfüllen die Anforderungen nach einem hohen Kₘ-Wert (geringe Affinität gegenüber Thrombin) und einer niedrigen Wechselzahl K_{cat}. Im Gegensatz dazu zeigen die Dextran-Peptid-Konjugate Dextran-D-CHG-Ala-Arg-pNA und Dextran-D-CHG-Gly-Arg-pNA deutlich geringere Kₘ-Werte (hohe Affinität gegenüber Thrombin) und eine hohe Wechselzahl K_{cat}. Bei Verwendung dieser beiden schnellen Substrate in einem Thrombingenerierungstest ist die Reaktionskinetik steiler als bei Verwendung des ungekoppelten Peptidsubstrats H-β-Ala-Gly-Arg-pNA oder des erfindungsgemäßen Substrats Dextran-β-Ala-Gly-Arg-pNA, und es kommt schnell zu einem Substratverbrauch, wodurch die Proportionalität von Substratumsatz und Thrombinkonzentration nicht mehr gewährleistet ist.

Die Herstellung eines erfindungsgemäßen Polysaccharid-Peptid-Konjugats kann durch ein beliebiges dem Fachmann bekanntes Verfahren erfolgen, das eine Bindung eines Peptids, das an seinem C-Terminus die Sequenz Ala-Gly-Arg-R enthält, wobei R eine durch Thrombin abspaltbare Signalgruppe ist, an das Polysaccharid erlaubt. Verfahren zur Herstellung von Polysaccharid-Peptid-Konjugaten sind z. B. in den Patentschriften US 6,011,008, WO 01/70272 A1 und US 6,949,524 B2 beschrieben. Bevorzugt sind Verfahren, bei denen ein aktiviertes Polysaccharid verwendet wird, das über amin-reaktive Gruppen verfügt. Vorteilhaft sind Verfahren, bei denen ein Polyaldehyd-Polysaccharid verwendet wird und wobei der Peptidanteil über Bildung einer Schiff'schen Base an das aktivierte Polysaccharid gekoppelt wird. Typischerweise ist zunächst eine Aktivierung des Polysaccharids notwendig, um amin-reaktive Gruppen, wie z. B. Aldehydgruppen, in dem Polysaccharid-Molekül zu generieren.

Besonders bevorzugt sind Verfahren, bei denen ein aktiviertes Polysaccharid verwendet wird, das über 40 bis 60, bevorzugterweise über 45 bis 55 Aldehygruppen pro Polysaccharid-Molekül verfügt. Die Generierung der Aldehydgruppen kann z. B. durch Oxidation mit geeigneten Oxidantien, wie Perjodsäure oder deren Salzen wie z. B. Natriumperiodat (NalO₄) erfolgen (siehe z. B. Hermanson, G. T., Bioconjugate Techniques, Academic Press 1996, Seiten 618-622) oder durch Verwendung alkylierender Substanzen, wie z. B. Glycidylether (siehe US 6,949,524 B2).

In einer bevorzugten Ausführungsform eines Verfahrens zur Herstellung eines erfindungsgemäßen Polysaccharid-Peptid-Konjugats wird ein aktiviertes Dextran einer molaren Masse von etwa 15.000 bis etwa 20.000 g/mol verwendet, das zur Generierung der Aldehydgruppen in einer 10%-igen Natriummetaperiodat-Lösung inkubiert wurde. Ein auf diese Weise aktiviertes Dextran enthält etwa 40 bis 55 Aldehydgruppen. Der Aktivierungsgrad kann photometrisch mit Hilfe der Purpald^{®}-Methode (Dickinson, R. G. und Jacobsen, N. W. (1970) A new sensitive and specific test for the detection of aldehydes: formation of 6-mercapto-3-substituted-s-triazolo[4,3-b]-s-tetrazines. J. Chem. Soc. D, 1719 - 1720) bestimmt werden. Nach einem säulenchromatographischen Aufreinigungsschritt, bei dem u. a. überschüssiges Natriummetaperiodat entfernt wird, und Umpufferung in Carbonat-Puffer (pH 8,5) wird zu dem aktivierten Dextran ein 7,5 bis 20fach, besonders bevorzugt ein 10fach molarer Überschuss an Peptid gegeben. Durch Zugabe einer reduzierenden Substanz zum Reaktionsansatz, wie z. B. von Natrium- oder Kaliumborhydrid, können die Schiff'schen Basen in stabilere, sekundäre Aminbindungen umgewandelt werden. Nach einem grössenausschlusschromatographischen Aufreinigungsschritt, bei dem u. a. ungebundenes Peptid entfernt wird, kann das Konjugat z. B. lyophilisiert werden. Bei derartig hergestellten Konjugaten sind etwa 7 bis 10 Peptidmoleküle an ein Dextranmolekül gebunden. Der Kopplungserfolg kann mittels HPLC-Analyse (High Performance Liquid Chromatography) überprüft werden.

Bevorzugt sind Polysaccharid-Peptid-Konjugate, worin pro Polysaccharid-Molekül mindestens 5, bevorzugt mindestens 10 Peptidmoleküle gebunden sind.

Weiterhin bevorzugt sind Polysaccharid-Peptid-Konjugate, bei denen der Peptidanteil über eine sekundäre Aminbindung an das Polysaccharid gebunden ist.

Ein weiterer Aspekt der vorliegenden Erfindung betrifft die Verwendung eines erfindungsgemäßen Polysaccharid-Peptid-Konjugats als Thrombinsubstrat in einem Verfahren zur Bestimmung der Thrombingenerierung. Aufgrund ihrer Molekülgröße von mindestens 10 kDa ist die Verwendung der erfindungsgemäßen Konjugate besonders dann vorteilhaft, wenn in dem Reaktionsansatz Thrombininhibitoren wie α₂-Makroglobulin vorhanden sind. Dies ist üblicherweise der Fall, wenn die Thrombingenerierung in Blut- oder Plasmaproben bestimmt wird. In einem typischen Verfahren zur Bestimmung der Thrombingenerierung wird eine Blut- oder Plasmaprobe eines Patienten mit dem Thrombinsubstrat vermischt und die Gerinnung wird durch Zugabe eines geeigneten Aktivators induziert. Aus der Messung der Menge an freigesetzter Signalgruppe über die Zeit wird eine Reaktionskinetik erstellt, die bei gesunden Personen nach einer anfänglichen Lag-Phase zunächst in eine exponentielle Phase der Thrombinbildung übergeht und schließlich mit zunehmender Inhibition des Thrombins eine Plateauphase erreicht. Eine besonders bevorzugte Verwendung eines erfindungsgemäßen Polysaccharid-Peptid-Konjugats ist die Verwendung als Thrombinsubstrat in einem Verfahren zur Bestimmung der Thrombingenerierung wie es z. B. in EP 420 332 B1 beschrieben ist.

### Figurenbeschreibung

**Fig. 1**

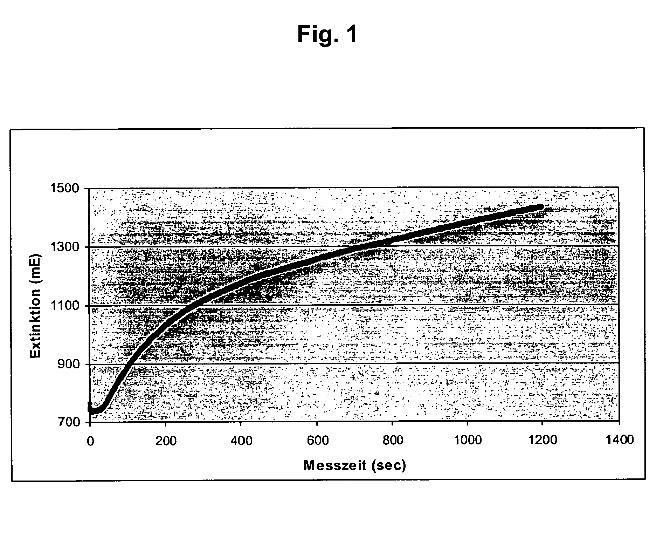

   Abbildung 1 zeigt eine Substratumsatzkurve mit dem ungekoppelten, kleinmolekularen Thrombinsubstrat β-Ala-Gly-Arg-pNA. Die Reaktionskurve geht nach Ablauf der Thrombinbildung und -inhibition in der Plasmaprobe in einen gleichmäßigen, stetigen und linearen Anstieg über, anstatt eine Plateauphase zu erreichen. Dieser Anstieg beruht auf der Spaltung des kleinmolekularen Peptidsubstrats durch α₂-Makroglobulin-gebundenes Thrombin.
**Fig. 2**

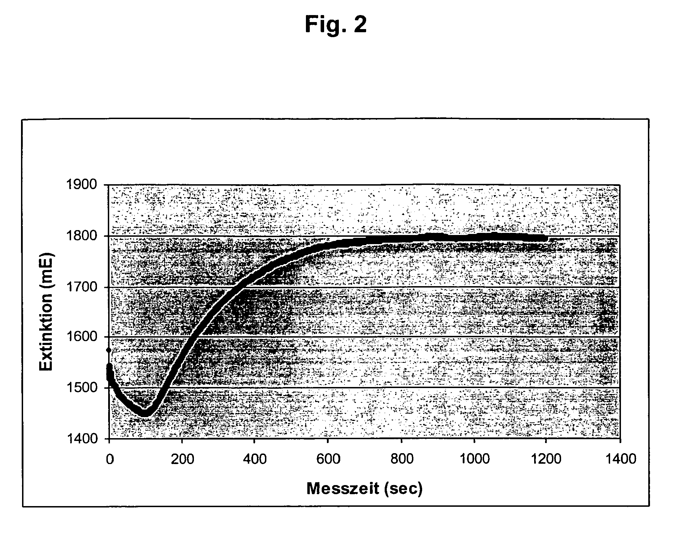

   Abbildung 2 zeigt eine Substratumsatzkurve mit dem makromolekularen Thrombinsubstrat Dextran-β-Ala-Gly-Arg-pNA. Die Reaktionskurve geht nach Ablauf der Thrombinbildung und -inhibition in der Plasmaprobe in einen Gleichgewichtszustand über und erreicht eine Plateauphase, in der kein Substratumsatz mehr stattfindet und die Extinktion konstant bleibt. Der Substratumsatz ist in diesem Fall direkt proportional zu der Menge an freiem Thrombin.
**Fig. 3**

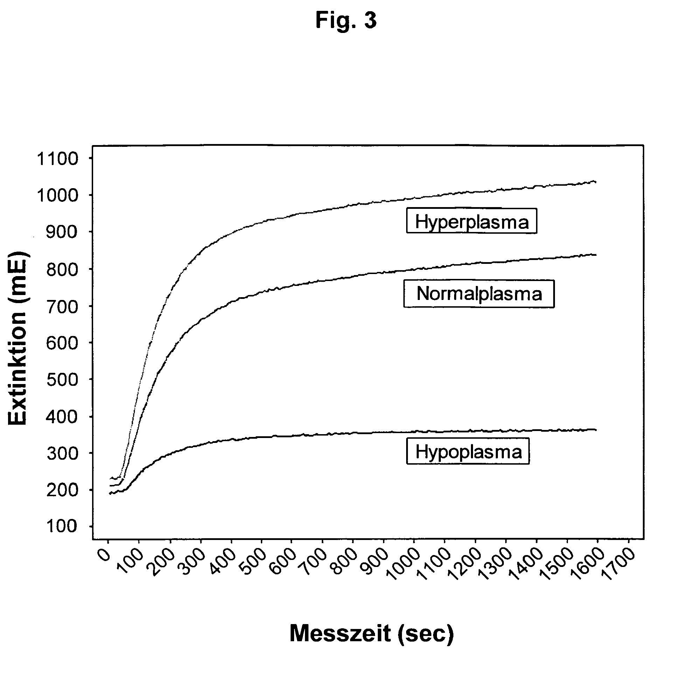

   Abbildung 3 zeigt die Substratumsatzkurve eines Normalplasmapools, eines Plasmapools mit erniedrigter Thrombinbildung (Hypopool) und eines Plasmapools mit erhöhter Thrombinbildung (Hyperpool). Der Reaktionskurvenverlauf und der Endpunkt der Extinktionänderung bei Reaktionsende ist abhängig von der Thrombinbildung in der Probe.

### Beispiele

### Beispiel 1: Herstellung des Dextran-gekoppelten Thrombinsubstrats Dextran-β-Ala-Gly-Arg-pNA

### a) Oxidation von Dextran

1500 mg Dextran mit einem Molekulargewicht von 15 bis 20 kDa laut Herstellerangaben (Fluka, Buchs, Schweiz) wurden in 30 ml einer 10%-igen Natriummetaperiodat-Lösung gelöst und 24 Stunden bei Raumtemperatur (19-26 °C) lichtgeschützt inkubiert. Überschüssiges Natriummetaperiodat und Nebenprodukte wurden durch Umpufferung in 0,1 M Natriumhydrogencarbonat-Puffer (pH 8,5) über PD-10 Fertigsäulen (GE Healthcare, Uppsala, Schweden) vom Reaktionsansatz abgetrennt.

### b) Kopplung von β-Ala-Gly-Arg-pNA an oxidiertes Dextran

Zu dem oxidierten Dextran (siehe Beispiel 1 a) wurde ein 10fach molarer Überschuss an β-Ala-Gly-Arg-pNA Peptid (Pefa 5134, Pentapharm, Basel, Schweiz) gegeben, und diese Lösung wurde 24 Stunden bei Raumtemperatur lichtgeschützt inkubiert. 15 min nach Reaktionsstart wurden pro ml Reaktionslösung 0,2 ml einer 1 M Natriumborhydrid-Lösung hinzugefügt. Die Reaktion wurde durch Zugabe von TRIS-Lösung (pH 8,0) mit einer Endkonzentration von 0,2 mol/l abgestoppt.

### c) Aufreinigung des Dextran-β-Ala-Gly-Arg-pNA Konjugats

Um nicht-gekoppelte Peptide bzw. freies pNA von dem gewünschten Dextran-β-Ala-Gly-Arg-pNA Konjugat abzutrennen, wurde das Konjugat durch Größenausschlusschromatographie über eine Sephacryl^{™} S-200 Säule (GE Healthcare, Uppsala, Schweden) in 0,1 % Essigsäure aufgereinigt. Die Ausbeute an Dextran-gekoppeltem Substrat betrug nach Lyophilisation 850 bis 1400 mg. Das so hergestellte Dextran-β-Ala-Gly-Arg-pNA Konjugat hatte ein Molekulargewicht von etwa 20 kDa.

Die Peptidsubstrate H-D-CHG-Ala-Arg-pNA (Pefa 5114, Pentapharm, Basel, Schweiz) und H-D-CHG-Gly-Arg-pNA (Pefa 081-04, Pentapharm, Basel, Schweiz) wurden auf die gleiche Weise an Dextran gekoppelt und nachfolgend aufgereinigt.

### Beispiel 2: Bestimmung des Aktivierungsgrades des oxidierten Dextrans

Zur Bestimmung des Aktivierungsgrades des oxidierten Dextrans wurde der Reaktionslösung, die nach Oxidation des Dextrans erhalten wurde (siehe Beispiel 1a) ein Aliquot entnommen und dieses mit 10 mM Natriumphosphat/300 mM Natriumchlorid-Puffer verdünnt. 100 µL der verdünnten Dextranlösung wurden mit 500 µl einer 1%-igen Purpald^{®}-Lösung in 1 N Natronlauge 45 min umgesetzt. Die Reaktion wurde anschließend durch Zugabe von 400 µl einer 2 mg/ml Natriumcyanoborhydrid-Lösung abgestoppt und die Absorption bei 540 nm gemessen. Die Aldehydkonzentration wurde durch Vergleich der Absorption mit einer Standardkurve ermittelt und der Aktivierungsgrad aus dem Quotienten Aldehydkonzentration/Dextrankonzentration berechnet.

Nach dem unter Beispiel 1a) beschriebenen Verfahren wurde oxidiertes Dextran mit einem Aktivierungsgrad von 40 bis 55 Aldehydgruppen pro Dextranmolekül erhalten.

### Beispiel 3: Bestimmung des Kopplungserfolges

Zur Bestimmung des Kopplungserfolges der unter Beispiel 1b) beschriebenen Kopplung von β-Ala-Gly-Arg-pNA an das oxidierte Dextran wurde nicht gebundenes Peptid vor und nach der Kopplung mittels HPLC-Analyse unter Verwendung einer Protein KW-803 Säule (Shodex, Japan) und durch Vergleich mit einer Peptid-Standardkurve quantifiziert. Aus der Mengendifferenz an freiem Peptid vor und nach der Kopplung wurde der gebundene Peptidanteil berechnet und dieser zur Dextranmenge ins Verhältnis gesetzt.

Pro Dextranmolekül waren 7 bis 10 Peptidmoleküle gebunden.

### Beispiel 4: Verwendung des erfindungsgemäßen Dextran-β-Ala-Gly-Arg-pNA Konjugats als Thrombinsubstrat zur Bestimmung des endogenen Thrombinpotenzials

Zur Bestimmung des endogenen Thrombinpotenzials wurde das lyophilisierte Thrombinsubstrat Dextran-β-Ala-Gly-Arg-pNA (siehe Bsp. 1) in 1 mL Tris-HCl Puffer [50 mM], pH 7,4 gelöst. Anschliessend wurden 135 µl defibriniertes plättchenarmes Plasma (PPP) mit 80 µl dieser Substratlösung bei 37 °C vorinkubiert. Die Thrombingenerierung wurde durch Zugabe von 30 µL Innovin® (Reagenz bestehend aus rekombinantem, humanem Gewebefaktor und einer Mischung von synthetischen Phospholipiden; Dade Behring Marburg GmbH, Deutschland) und 15 µL CaCl₂ [250 mM] gestartet. Gleichzeitig wurde die Messung gestartet. Die Extinktionsänderung wurde in einem Behring Coagulation System BCS®-System (Dade Behring Marburg GmbH, Marburg, Deutschland) bei einer Wellenlänge von λ= 405 nm für mindestens 20 Minuten verfolgt (siehe Fig. 2).

Zu Vergleichszwecken wurde die Bestimmung des endogenen Thrombinpotenzials parallel mit dem ungekoppelten Substrat H-β-Ala-Gly-Arg-pNA durchgeführt. Die dazu verwendete Substratlösung enthielt 1 mM H-β-Ala-Gly-Arg-pNA (siehe Fig. 1).

## Patentansprüche

1. Polysaccharid-Peptid-Konjugat mit einem Molekulargewicht von mehr als 10 kDa **dadurch gekennzeichnet, dass** der Peptidanteil an seinem C-Terminus die Sequenz Ala-Gly-Arg-R enthält, wobei R eine durch Thrombin abspaltbare Signalgruppe ist.

2. Polysaccharid-Peptid-Konjugat gemäß Anspruch 1 **dadurch gekennzeichnet, dass** der Peptidanteil insgesamt aus einer Sequenz von 3 bis 5 Aminosäureresten besteht, bevorzugt aus nicht mehr als insgesamt 8 Aminosäureresten.

3. Polysaccharid-Peptid-Konjugat gemäß den Ansprüchen 1 und 2 **dadurch gekennzeichnet, dass** zur Herstellung des Konjugats ein Polysaccharid verwendet wurde, das aus Monosaccharid-Einheiten aufgebaut ist, welche vicinale Diole oder Hydroxyl/Aminogruppen oder Hydroxyl/Carbonylgruppen oder Carbonyl/Carbonylgruppen enthalten.

4. Polysaccharid-Peptid-Konjugat gemäß den Ansprüchen 1 bis 3 **dadurch gekennzeichnet, dass** der Polysaccharidanteil aus Dextran besteht.

5. Polysaccharid-Peptid-Konjugat gemäß einem der Ansprüche 1 bis 4 **dadurch gekennzeichnet, dass** der Polysaccharidanteil eine molare Masse von etwa 10.000 bis etwa 40.000 g/mol, bevorzugt von etwa 12.000 bis etwa 20.000 g/mol, besonders bevorzugt von etwa 15.000 g/mol hat.

6. Polysaccharid-Peptid-Konjugat gemäß einem der Ansprüche 1 bis 5 **dadurch gekennzeichnet, dass** die abspaltbare Signalgruppe R eine chromogene Gruppe ist.

7. Polysaccharid-Peptid-Konjugat gemäß Anspruch 6 **dadurch gekennzeichnet, dass** die chromogene Gruppe para-Nitroanilin (pNA) ist.

8. Polysaccharid-Peptid-Konjugat gemäß einem der Ansprüche 1 bis 5 **dadurch gekennzeichnet, dass** die abspaltbare Signalgruppe R eine fluorogene Gruppe ist.

9. Polysaccharid-Peptid-Konjugat gemäß einem der Ansprüche 1 bis 8 **dadurch gekennzeichnet, dass** pro Polysaccharid-Molekül mindestens 5, bevorzugt mindestens 10 Peptidmoleküle gebunden sind.

10. Polysaccharid-Peptid-Konjugat gemäß einem der Ansprüche 1 bis 9 **dadurch gekennzeichnet, dass** der Peptidanteil über eine sekundäre Aminbindung an das Polysaccharid gebunden ist.

11. Verwendung eines Polysaccharid-Peptid-Konjugats gemäß einem der Ansprüche 1-10 in einem Verfahren zur Bestimmung der Thrombingenerierung in einer Probe.
